(19) Europäisches Patentamt
European Patent Office
Office européen des brevets

(11) Veröffentlichungsnummer: **0 082 364**
**B1**

# (12) EUROPÄISCHE PATENTSCHRIFT

(45) Veröffentlichungstag der Patentschrift: **23.04.86**

(51) Int. Cl.⁴: **C 07 D 233/22**, A 61 K 31/415

(21) Anmeldenummer: **82111097.0**

(22) Anmeldetag: **01.12.82**

## (54) (+)-2-(1-(2,6-Dichlorphenoxy)-ethy1)-1,3-diazacyclopent-2-en, dessen Herstellung und seine Verwendung in pharmazeutischen Präparaten.

(30) Priorität: **10.12.81 DE 3149010**

(43) Veröffentlichungstag der Anmeldung: **29.06.83 Patentblatt 83/26**

(45) Bekanntmachung des Hinweises auf die Patenterteilung: **23.04.86 Patentblatt 86/17**

(84) Benannte Vertragsstaaten: **AT BE CH DE FR GB IT LI LU NL SE**

(56) Entgegenhaltungen:
**DE-A-1 695 555**
**DE-A-1 795 843**
**DE-A-1 935 479**
**DE-A-2 818 367**

**J.MARCH: "Advanced organic chemistry: reactions, mechanisms, and structure", International Student Edition, 1968, McGraw-Hill Book Company, New York (USA);**

(73) Patentinhaber: **A. Nattermann & Cie. GmbH, Nattermannallee 1, D-5000 Köln 30 (DE)**

(72) Erfinder: **Biedermann, Jürgen, Dr., Kirschenweg 14, D-5024 Pulheim-Stommeln (DE)**
Erfinder: **Prop, Gerrit, Dr., Mohnblumenweg 25, D-5024 Pulheim (DE)**
Erfinder: **Doppelfeld, Ille-Stephanie, Im Bruchfeldchen 35, D-5151 Bergheim-Glessen (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

**EP 0 082 364 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft (+)-2-[1-(2,6-Dichlorphenoxy)- ethyl]- 1,3-diazacyclopent-2-en der Formel I

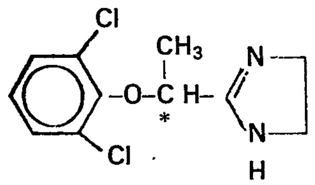

(I)

* = Asymmetriezentrum

und seine pharmazeutisch verträglichen Säureadditionssalze, Verfahren zur Herstellung dieser Verbindung, sowie die Verwendung als Wirkstoff in Arzneimitteln mit wertvollen pharmakologischen Eigenschaften und zwar in erster Linie therapeutischen Wirkungen auf das Zentralnervensystem.

Herstellungsverfahren des Racemats (±)-2-[1-(2,6-Dichlorphenoxy)-ethyl]-1,3-diazacyclopent-2-en, das als Antihypertonikum bekannt ist, enthalten die Offenlegungsschriften DE-OS 16 95 555 und DE-OS 19 35 479 sowie die Patentschrift DE-PS 17 95 843.

Obwohl es bekannt war, dass 2-[1-(2,6-Dichlorphenoxy) -ethyl] -1,3-diazacyclopent-2-en wegen des asymmetrisch substituierten Kohlenstoffatoms in zwei optisch aktiven enantiomeren Formen vorliegen kann, wurde eine Trennung bzw. Herstellung der optisch aktiven links- bzw. rechtsdrehenden Enantiomeren bisher nicht durchgeführt.

Die Abtrennung des rechtsdrehenden Enantiomeren zur Aufklärung der Zusammenhänge zwischen optischer Aktivität und biologischer Wirksamkeit gelang durch die an sich bekannte Racematspaltungs-Methode mit Hilfe der rechtsdrehenden Dibenzoylweinsäure.

Die pharmakologischen Untersuchungen des rechtsdrehenden Enantiomeren ergaben überraschenderweise zentraldämpfende Eigenschaften, während die blutdrucksenkende Wirkung nicht mehr auftrat.

Die Synthese der erfindungsgemässen optisch aktiven Verbindung ist dadurch gekennzeichnet, dass sie von optisch aktiven Ausgangssubstanzen ausgeht. So wird z.B. ein (+)-2-Hydroxypropionsäure-$C_{1-4}$-Alkylester mit überschüssigem Thionylchlorid in Gegenwart katalytischer Mengen von Dimethylformamid unter Konfigurationsumkehr zum (−)-2-Chlorpropionsäure-$C_{1-4}$-Alkylester umgesetzt. Die anschliessende Veretherung mit 2,6-Dichlorphenol erfolgt unter erneuter Konfigurationsumkehr in geeigneten organischen Lösungsmitteln, wie z.B. Acetonitril, Butanon, Dimethylformamid in Gegenwart von Hilfsbasen, wie z.B. Natriumhydrid, Alkalimethylat, Alkaliethylat oder 1,4-Diazabicyclo-(2,2,2) -octan im Temperaturbereich von 60–150 °C, vorzugsweise bei 80 °C zum $C_{1-4}$-Alkylester der (+)-2-(2,6-Dichlorphenoxy) -propionsäure, die neben ihren funktionellen Säurederivaten als Ausgangssubstanz für die Synthese von 1,3-Diazacyclopenten-Derivaten verwendet wird. Dabei kann die Umsetzung mit Ethylendiamin selbst oder mit einem reaktiven N-Derivat des Ethylendiamins oder mit Ammoniak bzw. ammoniakabgebenden Mitteln und einer in das Ethylendiamin durch Behandlung mit Ammoniak überführbaren Verbindung durchgeführt werden.

Als funktionelle Säurederivate können z.B. $C_{1-4}$-Alkylester, Säurehalogenide, Amide, Thiamide, Amidine, Imidsäureester oder das Nitril der 2-(2,6- Dichlorphenoxy) - propionsäure verwendet werden.

Im erfindungsgemässen Verfahren wird (+)-2-(2,6-Dichlorphenoxy) -propionsäureethylester mit einem grossen Überschuss an 1,2-Diaminoethan bei Raumtemperatur zum (+)-2- (2,6-Dichlorphenoxy) -propionsäure-N- (2-aminoethyl)-amid umgesetzt, das seinerseits mit einem Titantetrachlorid/Tetrahydrofuran-Komplex in Chloroform in Gegenwart von 4-Dimethylaminopyridin bei 0–30 °C zum (+)-2- [1-(2,6-Dichlorphenoxy) -ethyl]- 1,3-diazacyclopent-2-en dehydratisiert bzw. cyclisiert wird.

Die anschliessende Überführung der rechtsdrehenden Base in ein physiologisch unbedenkliches Säureadditionssalz erfolgt mit einer geeigneten anorganischen oder organischen Säure in einem niederen Alkohol. So erhält man aus der (+)-Base z.B. mit Propanol-2/Chlorwasserstoff das (+)-2-[1-(2,6-Dichlorphenoxy)- ethyl]-1,3-diazacyclopenten-2-hydrochlorid.

Ein anderes Verfahren geht ebenfalls von (+)-2-(2,6-Dichlorphenoxy) -propionsäureethylester aus, der bei Raumtemperatur mit einer ethanolischen Lösung von Ammoniak zum in Ethanol schwerlöslichen (−)-2- (2,6-Dichlorphenoxy) -propionsäureamid umgesetzt wird. Dieses Amid wird bei 0–30 °C mit einem Titantetrachlorid/Tetrahydrofuran-Komplex und 4-Methylmorpholin in Chloroform zum (+)-2- (2,6-Dichlorphenoxy) -propionsäurenitril dehydratisiert. Man überführt sodann das Nitril mit Ethanol in Chloroform durch Einleiten von Chlorwasserstoff bei 0 °C in das (+)-2- (2,6-Dichlorphenoxy) -propionimidsäureethylesterhydrochlorid, löst dieses in Ethanol und cyclisiert durch Zugabe von 1,2-Diaminoethan zum (+)-2-[1-(2,6-Dichlorphenoxy) -ethyl]-1,3-diazacyclopent-2-en. Von der so erhaltenen (+)-Base wird analog dem vorangehenden Verfahren das (+)-2-[1-(2,6-Dichlorphenoxy) -ethyl]-1,3-diazacyclopenten-2-hydrochlorid hergestellt.

Die Racematspaltung des (±)-2- [1-(2,6-Dichlorphenoxy) -ethyl]-1,3-diazacyclopent-2-ens erfolgt in an sich bekannter Weise nach den in der organischen Chemie üblichen Verfahren (Racematspaltung: vergl. Übersicht in Houben-Weyl, Methoden der organischen Chemie, Bd. 4/2, 505 (1955), Georg Thieme Verlag, Stuttgart). Man überführt die racemische Base in einem geeigneten Lösungsmittel, wie z.B. Aceton, mit der optisch aktiven (+)-Dibenzoylweinsäure in zwei durch ihre Löslichkeit von einander unterschiedliche Diastereomeren-Salzpaare. Das wegen seiner geringen Löslichkeit in Aceton auskristallisierte Salz, in dem die (+)-Base (+)-2-[1-(2,6-Dichlorphenoxy) -ethyl] -1,3-diazacyclopent-2-en stark angereichert als (+)-Dibenzoyltartrat vorliegt, wird abgesaugt, die (+)-Base mit gesättigter Natriumcarbonatlösung freigesetzt und erneut mit (+)-Dibenzoylweinsäure in Aceton versetzt. Das auf diese Weise erhaltene 2. Kristallisat liegt als (+)-Dibenzoyltartrat der mittlerweile hochangereicherten (+)-Base vor und wird durch zweimalige Kristallisation aus Ethanol weiter gereinigt. Mit Natriumcarbonatlösung wird die (+)-Base freigesetzt und mit Propanol-(2)/Chlorwasserstoff in das optisch reine (+)-2-[1-(2,6-Dichlorphenoxy) -ethyl]- 1,3-diazacyclopenten-2-hydrochlorid überführt.

Aus der freien Base der erfindungsgemässen Verbindung können durch Behandlung mit geeigneten Säuren die verschiedensten pharmazeutisch verträglichen Säureadditionssalze nach üblichen Methoden hergestellt werden. Für die Herstellung derartiger Salze kommen anorganische Säuren, z.B. Bromwasserstoffsäure, Schwefelsäure oder Phosphorsäure, oder organische Säuren, wie z.B. Essigsäure, Glykolsäure, Bernsteinsäure, Fumarsäure, Äpfelsäure, Weinsäure, Citronensäure, Benzoesäure oder Zimtsäure, insbesondere und bevorzugt Chlorwasserstoffsäure in Frage.

Die erfindungsgemässen Verbindungen haben wertvolle pharmakologische Eigenschaften, und zwar in erster Linie therapeutische Wirkungen auf das Zentralnervensystem, und können als Wirkstoffe von krampfhemmenden bzw. sedierend wirkenden Arzneimitteln zur Anwendung kommen.

Die Erfindung betrifft ebenfalls Präparate, welche die Verbindung der Formel I oder pharmazeutisch verwendbare Säureadditionssalze dieser Verbindung enthalten. Bei den erfindungsgemässen pharmazeutischen Präparaten handelt es sich um solche zur enteralen, wie oralen oder rektalen sowie parenteralen Verabreichung, welche den pharmakologischen Wirkstoff allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen. Die Dosierung der Verbindung liegt üblicherweise zwischen 0,05 und 50 mg je Dosis, vorzugsweise bei 0,075–0,1 mg je Dosis und kann ein- oder mehrmals verabreicht werden.

Die Herstellung der erfindungsgemässen Verbindungen wird durch die folgenden Beispiele näher erläutert. Die angegebenen Schmelzpunkte wurden mit einem Büchi 510-Schmelzpunktbestimmungsapparat gemessen und sind in °C angegeben und nicht korrigiert.

Beispiel 1

Synthese von (+)-2-[1- (2,6-Dichlorphenoxy) -ethyl] -1,3-diazacyclopenten-(2)-hydrochlorid.

(−)-2-Chlorpropionsäureethylester.

260 g (≙ 2,202 Mol) (+)-2-Hydroxypropionsäureethylester $[\alpha]_D^{20}$ = +11,3° (unverdünnt) werden mit 274,0 g (≙ 2,303 Mol) Thionylchlorid in Gegenwart von 1,5 ml Dimethylformamid in der Siedehitze chloriert. Man erhält 90,0 g (−)-2-Chlorpropionsäureethylester $C_5H_9ClO_2$ [136,6] Kp: 142–143 °C

$[\alpha]_D^{20}$ = −19,4° (unverdünnt)

(+)-2-(2,6-Dichlorphenoxy) -propionsäureethylester.

70,0 g (≙ 0,429 Mol) 2,6-Dichlorphenol werden in 300 ml Butanon mit 40,6 g (≙ ,579 Mol) Kaliummethylat und 81,0 g (≙ 0,593 Mol) (−)-2-Chlorpropionsäureethylester 48 Stunden unter Rühren und Rückfluss zum Sieden erhitzt. Nach Aufarbeitung erhält man 64,0 g (+)-2-(2,6-Dichlorphenoxy) -propionsäureethylester $C_{11}H_{12}Cl_2O_3$ [263,1], $Kp_{0,1}$: 111–112 °C

$[\alpha]_D^{20}$ = +36,8° (unverdünnt)

(+)-2-(2,6-Dichlorphenoxy) -propionsäure-N- (2-aminoethyl)-amid. 42,0 g (≙ 0,160 Mol) (+)-2- (2,6-Dichlorphenoxy) -propionsäureethylester werden mit 194,0 g (≙ 3,228 Mol) 1,2-Diaminoethan 6 Stunden bei Raumtemperatur gerührt und ergeben nach Aufarbeitung 34,0 g (+)-2-(2,6-Dichlorphenoxy)-propionsäure-N- (2-aminoethyl)-amid, $C_{11}H_{14}Cl_2N_2O_2$ [277,2] als hochviskoses Öl,

$[\alpha]_D^{20}$ = +5,6° (c=l/Ethanol)

(+)-2- [1-(2,6-Dichlorphenoxy)-ethyl]-1,3-diazacyclopenten-(2). 50,2 g (≙ 0,2646 Mol) Titantetrachlorid werden bei 0 °C in einer Mischung aus 700 ml Chloroform abs. und 25 ml Tetrahydrofuran gelöst und mit 30,0 g (≙ 0,108 Mol) (+)-2- (2,6-Dichlorphenoxy) -propionsäure-N- (2-aminoethyl)-amid versetzt. Anschliessend lässt man sehr langsam bei 0 °C unter Rühren eine Lösung von 63,0 g (=0,5155 Mol) 4-Dimethylaminopyridin zutropfen und den Ansatz nach Beendigung der Zugabe noch 36 Stunden bei Raumtemperatur weiterrühren. Nach Aufarbeitung und säulenchromatographischer Reinigung (Kieselgel-Trockensäule, Elutionsmittel: Chloroform/Tetrahydrofuran 3:1) erhält man 16,0 g (+)-2-[1-(2,6-Dichlorphenoxy) -ethyl]-1,3-diazacyclopenten-(2) $C_{11}H_{12}Cl_2N_2O$ [259,1], Fp. 127–128 °C

$[\alpha]_D^{20}$ = +80,6° (c=l/Ethanol)

(+)-2-[1-(2,6-Dichlorphenoxy) -ethyl] -1,3-diazacyclopenten-(2)- hydrochlorid.

10,0 g (+)-2- [1-(2,6-Dichlorphenoxy) -ethyl] -1,3-diazacyclopenten-(2) werden in 40 ml Propanol-(2) gelöst und mit 40 ml einer gesättigten Lösung von Chlorwasserstoff in Propanol-(2)

versetzt. Man erhält nach entsprechender Aufarbeitung 7,8 g (+)-2-[1- [2,6-Dichlorphenoxy) - ethyl]- 1,3-diazacyclopenten-(2)-hydrochlorid $C_{11}H_{12}Cl_2N_2O \cdot HCl$ [295,6], Fp. 229 °C

$[\alpha]_D^{20}$ = +33,4° (c=I/Ethanol)

Beispiel 2

Synthese von (+)-2-[1- (2,6-Dichlorphenoxy) -ethyl] -1,3-diazacyclopenten-(2)-hydrochlorid.

Die Ausgangssubstanz ist in Beispiel 1, Stufe 2, beschrieben. (−)-2- (2,6-Dichlorphenoxy)-propionsäureamid.

In eine Lösung von 55 g (≙ 0,2091 Mol) (+)-2-(2,6-Dichlorphenoxy) -propionsäureethylester in 100 ml mit Ammoniak gesättigtem Ethanol wird 24 Stunden lang Ammoniak unter Rühren bei Raumtemperatur eingeleitet. Nach Aufarbeitung erhält man 35,0 g (−)-2(2,6-Dichlorphenoxy) -propionsäureamid $C_9H_9Cl_2NO_2$ [234,1], Fp. 192 °C

$[\alpha]_D^{20}$ = −20,3° (c=I/Aceton)

(+)-2-(2,6-Dichlorphenoxy)-propionitril.

Eine Lösung von 17,9 g (≙ 0,0944 Mol) Titantetrachlorid in 50 ml Chloroform abs. und 7 ml Tetrahydrofuran wird mit 11,0 g (≙ 0,0470 Mol) (−)-2- (2,6-Dichlorphenoxy)- propionsäureamid versetzt. Anschliessend lässt man sehr langsam unter Rühren bei 0 °C eine Lösung von 19,0 g (≙ 0,1877 Mol) 4-Methylmorpholin eintropfen und den Kolbeninhalt nach der Beendigung der Zugabe noch 24 Stunden bei 30 °C weiterrühren. Nach Aufarbeitung erhält man 7,5 g (+)-2- (2,6-Dichlorphenoxy) -propionitril $C_9H_7Cl_2NO$ [216,1], $Kp_{0,1}$: 81 °C

$[\alpha]_D^{20}$ = +76° (unverdünnt)

(+)-2- [1-(2,6-Dichlorphenoxy) -ethyl] -1,3-diazacyclopenten-(2).

22,0 g (≙ 0,1018 Mol) (+)-2- (2,6-Dichlorphenoxy) -propionitril werden bei 0 °C mit 4,7 g (≙ 0,1018 Mol) Ethanol in 50 ml Chloroform. abs. durch Einleiten von Chlorwasserstoff in das (+)-2-(2,6-Dichlorphenoxy)- propionimidsäureethylester- hydrochlorid überführt. Dieses wird nach dem Abziehen des Lösungsmittels im Vakuum bei 20 °C anschliessend in 20 ml Ethanol gelöst und mit 6,6 g (≙ 0,11 Mol) 1,2-Diaminoethan zum (−)-2- [1-(2,6-Dichlorphenoxy)-ethyl] -1,3-diazacyclopenten-(2) $C_{11}H_{12}Cl_2N_2O$ [259,1] cyclisiert. Man erhält nach Aufarbeitung 17 g Produkt. Fp. 128 °C

$[\alpha]_D^{20}$ = +80,4° (c=I/Ethanol)

(+)-2-[1-(2,6-Dichlorphenoxy) -ethyl) -1,3-diazacyclopenten-(2)- hydrochlorid.

4,0 g (+)-2-[1-(2,6-Dichlorphenoxy)-ethyl]-1,3-diazacyclopenten-(2) werden in 16 ml Propanol-(2) gelöst und mit 16 ml einer gesättigten Lösung von Chlorwasserstoff in Propanol-(2) versetzt. Man erhält 3,3 g (+)-2-[ 1-(2,6-Dichlorphenoxy) -ethyl] -1,3-diazacyclopenten-(2)-hydrochlorid $C_{11}H_{12}Cl_2N_2O \cdot HCl$ [295,6], Fp. 229 °C

$[\alpha]_D^{20}$ = +33,2° (c=I/Ethanol)

Beispiel 3

Gewinnung von (+)-2-[1-(2,6-Dichlorphenoxy) -ethyl] -1,3-diazacyclopent-(2)-en durch Racematspaltung von (±)-2- [1-(2,6-Dichlorphenoxy) -ethyl] -1,3-diazacyclopent-2-en mit (+)-Dibenzoylweinsäure.

10 g (±)-2- [1-(2,6-Dichlorphenoxy)-ethyl]-1,3-diazacyclopent-(2)-en werden bei Raumtemperatur in 400 ml Aceton gelöst, unter Rühren zu einer Lösung von 14,5 g (+)-Dibenzoylweinsäure $[\alpha]_D^{20}$ = +110° (c=I/Ethanol 96%) in 2000 ml Aceton gegeben und ohne Rühren 24 Stunden bei 21 °C stehen gelassen. Das ausgefallene Salz (12,8 g) liegt als (+)-Dibenzoyltartrat der angereicherten (+)-Base vor [$[\alpha]_D^{20}$ = +65,6° (c=I/Ethanol)]. Aus diesem Salz erhält man nach Neutralisation mit gesättigter Natriumcarbonatlösung und Extraktion mit Dichlormethan 6,9 g Base, die nach Lösen in 550 ml Aceton erneut mit einer Lösung von 10,0 g (+)-Dibenzoylweinsäure in 2200 ml Aceton versetzt wird. Nach 24 Stunden erhält man 9,8 g des (+)-Dibenzoyltartrates der jetzt bereits hoch angereicherten (+)-Base [$[\alpha]_D^{20}$ = +67,5° (c=I/Ethanol)]. Die weitere Reinigung erfolgt durch Umkristallisation von 9,7 g dieses Salzes aus 250 ml 60 °C heissem Ethanol. Nach 24 Stunden (21 °C) erhält man 4,6 g (+)-Dibenzoyltartrat der(+)-Base [$[\alpha]_D^{20}$ = +72,6° (c=I/Ethanol)]. Die 2. Umkristallisation von 4,5 g dieses Salzes aus 70 ml 60 °C heissem Ethanol ergibt nach 24 Stunden (21 °C) 2,9 g (+)-2-[1- (2,6-Dichlorphenoxy)-ethyl] -1,3-diazacyclopenten-(2)-(+)-dibenzoyltartrat, [$[\alpha]_D^{20}$ = +77,8° (c=I/Ethanol)].

Aus 2,8 g des letzten Salzes werden mit 15 ml gesättigter Natriumcarbonatlösung und 40 ml Dichlormethan 1,3 g reine (+)-Base freigesetzt, die in 2,5 ml Propanol-(2) gelöst und mit 2,5 ml einer gesättigten Lösung von Chlorwasserstoff in Propanol-(2) versetzt und in das Hydrochlorid überführt wird. Man erhält 0,9 g (+)-2-[1-(2,6-Dichlorphenoxy) -ethyl] -1,3-diazacyclopenten-(2)- hydrochlorid $C_{11}H_{12}Cl_2N_2O \cdot HCl$ [295,6], Fp. 228–229 °C $[\alpha]_D^{20}$ = +33,4° (c=I/Ethanol)

**Patentansprüche für die Vertragsstaaten BE, CH, DE, FR, GB, IT, LI, LU, NL, SE**

1. (+)-2-[1- (2,6-Dichlorphenoxy) -ethyl]-1,3-diazacyclopent-2-en und seine pharmazeutisch verträglichen Säureadditionssalze.

2. (+)-2-[1- (2,6-Dichlorphenoxy) -ethyl]-1,3-diazacyclopenten-2- hydrochlorid.

3. Verfahren zur Herstellung von (+)-2-[1-(2,6-Dichlorphenoxy) -ethyl] -1,3-diazacyclopent-2-en gemäss den Ansprüchen 1–2, dadurch gekennzeichnet, dass man (+)-2-Hydroxypropionsäure-$C_{1-4}$- Alkylester mit Thionylchlorid in Gegenwart katalytischer Mengen von Dimethylformamid unter Konfigurationsumkehr zum (–)-2-Chlorpropionsäure-$C_{1-4}$- Alkylester umsetzt, der bei der Veretherung mit 2,6-Dichlorphenol unter erneuter Konfigurationsumkehr in einem geeigneten organischen Lösungsmittel in Gegenwart von Hilfsbasen im Temperaturbereich von 60–150 °C in den (+)-2- (2,6-Dichlorphenoxy)-propionsäure-$C_{1-4}$-Alkylester überführt wird, der nach an sich bekannten Methoden das (+)-1,3-Diazacyclopenten-Derivat ergibt.

4. Verfahren zur Herstellung von (+)-2-[1-(2,6-Dichlorphenoxy) -ethyl] -1,3-diazacyclopent-2-en gemäss den Ansprüchen 1–2, durch die an sich bekannte Racematspaltungsmethode über die diastereomeren Salze mit Hilfe optisch aktiver Säuren und anschliessender Freisetzung des Enantiomeren, dadurch gekennzeichnet, dass man als optisch aktive Säure rechtsdrehende Dibenzoylweinsäure verwendet.

5. Pharmazeutische Präparate, dadurch gekennzeichnet, dass sie eine Verbindung gemäss den Ansprüchen 1–2 als Wirkstoff im Gemisch mit üblichen pharmazeutischen Hilfs- und Trägerstoffen enthalten.

**Patentansprüche für den Vertragsstaat AT**

1. Verfahren zur Herstellung von (+)-2-[1-(2,6-Dichlorphenoxy) -ethyl] -1,3-diazacyclopent-2-en und seinen pharmazeutisch verträglichen Säureadditionssalzen, dadurch gekennzeichnet, dass man (+)-2-Hydroxypropionsäure-$C_{1-4}$-Alkylester mit Thionylchlorid in Gegenwart katalytischer Mengen von Dimethylformamid unter Konfigurationsumkehr zum (–)-2-Chlorpropionsäure-$C_{1-4}$-Alkylester umsetzt, der bei der Veretherung mit 2,6-Dichlorphenol unter erneuter Konfigurationsumkehr in einem geeigneten organischen Lösungsmittel in Gegenwart von Hilfsbasen im Temperaturbereich von 60–150 °C in den (+)-2-(2,6-Dichlorphenoxy) -propionsäure-$C_{1-4}$-Alkylester überführt wird, der nach an sich bekannten Methoden das (+)-1,3-Diazacyclopenten-Derivat ergibt.

2. Verfahren zur Herstellung von (+)-2-[1-(2,6-Dichlorphenoxy)-ethyl] -1,3-diazacyclopent-2-en und seinen pharmazeutisch verträglichen Säureadditionssalzen durch die an sich bekannte Racematspaltungsmethode über die diastereomeren Salze mit Hilfe optisch aktiver Säuren und anschliessender Freisetzung des Enantiomeren, dadurch gekennzeichnet, dass man als optisch aktive Säure rechtsdrehende Dibenzoylweinsäure verwendet.

**Revendications pour les Etats contractants: BE, CH, DE, FR, GB, IT, LI, LU, NL, SE**

1. Le (+)-2- [1-(2,6-dichlorophénoxy)-éthyl]-1,3-diazacyclopenta-2-ène et ses sels formés par addition avec des acides acceptables pour l'usage pharmaceutique.

2. Le chlorhydrate du (+)-2-[ 1-(2,6-dichlorophénoxy)- éthyl] -1,3-diazacyclopenta-2-ène.

3. Procédé de préparation du (+)-2- [1-(2,6-dichlorophénoxy) -éthyl] -1,3-diazacyclopenta-2-ène selon les revendications 1 et 2, caractérisé en ce que l'on fait réagir un (+)-2-hydroxypropionate d'alkyle en C1–C4 avec le chlorure de thionyle en présence de quantités catalytiques de diméthylformamide avec inversion de la configuration en le (–)-2-chloropropionate d'alkyle en C1–C4 qu'on convertit lui-même, par éthérification à l'aide du 2,6-dichlorophénol, avec nouvelle inversion de configuration, dans un solvant organique approprié, en présence de bases auxiliaires, dans l'intervalle de température de 60 à 150 °C, en le (+)-2- (2,6-dichlorophénoxy) -propionate d'alkyle en C1–C4 qui donne par des procédés connus en soi le dérivé du (+)-1,3-diazacyclopentène.

4. Procédé de préparation du (+)-2- [1-(2,6-dichlorophénoxy) -éthyl] -1,3-diazacyclopenta-2-ène selon les revendications 1 et 2, par le procédé connu en soi de résolution des racémates par l'intermédiaire des sels diastéréoisomères à l'aide d'acides énantiomères, avec libération subséquente de l'énantiomère, caractérisé en ce que l'on utilise en tant qu'acide énantiomère l'acide dibenzoyltartrique dextrogyre.

5. Compositions pharmaceutiques caractérisées en ce qu'elles contiennent un composé selon les revendications 1 et 2 en tant que substance active en mélange avec des produits auxiliaires et véhicules pharmaceutiques usuels.

**Revendications pour l'Etat contractant: AT**

1. Procédé de préparation du (+)-2- [1-(2,6-dichlorophénoxy) -éthyl] -1,3-diazacyclopenta-2-ène et de ses sels formés par addition avec des acides acceptables pour l'usage pharmaceutique, caractérisé en ce que l'on convertit un (+)-2-hydroxypropionate d'alkyle en C1-C4, à l'aide du chlorure de thionyle, en présence de quantités catalytiques de diméthylformamide, avec inversion de configuration, en le (–)-2-chloropropionate d'alkyle en C1–C4, qu'on convertit lui-même par éthérification à l'aide du 2,6-dichlorophénol, avec nouvelle inversion de la configuration, dans un solvant organique approprié, en présence de bases auxiliaires, dans l'intervalle de température de 60 à 150 °C, en le (+)-2-(2,6-dichlorophénoxy) -propionate d'alkyle en C1–C4, lequel donne par des procédés connus en soi le dérivé de (+)-1,3-diazacyclopentène.

2. Procédé de préparation du (+)-2-[1-(2,6-dichlorophénoxy) -éthyl] -1,3-diazacyclopenta-2-ène et de ses sels formés par addition avec des

acides acceptables pour l'usage pharmaceutique par le procédé connu en soi de résolution des racémates par l'intermédiaire des sels diastéréoisomères à l'aide d'acides énantiomères avec libération subséquente de l'énantiomère, caractérisé en ce que l'on utilise en tant qu'acide énantiomère l'acide dibenzoyltartrique dextrogyre.

**Claims for the contracting States: BE, CH, DE, FR, GB, IT, LI, LU, NL, SE**

1. (+)-2-[1- (2,6-Dichlorophenoxy) -ethyl] -1,3-diazacyclopent-2-ene and the pharmaceutically acceptable acid addition salts thereof.

2. (+)-2-[1- (2,6-Dichlorophenoxy) -ethyl] -1,3-diazacyclopent-2-ene hydrochloride.

3. A process for the production of (+)-2-[1-(2,6-dichlorophenoxy) -ethyl] -1,3-diazacyclopent-2-ene according to claims 1 to 2, characterised in that a (+)-2-hydroxypropionic acid-$C_{1-4}$ alkylester is reacted with thionylchloride in the presence of catalytic quantities of dimethylformamide with configuration reversal to produce a (−)-2-chloropropionic acid-$C_{1-4}$-alkyl ester which is converted into (+)-2-(2,6-dichlorophenoxy)-propionic acid-$C_{1-4}$ alkyl ester with a further configuration reversal, by etherification with 2,6-dichlorophenol in the presence of a base at a temperature in the range of from 60 to 150 °C, in a suitable organic solvent, this alkylester being used for the production of the (+)-1,3-diazacyclopentene derivative according to known methods.

4. A process for the production of (+)-2-[1-(2,6-dichlorophenoxy) -ethyl] -1,3-diazacyclopent-2-ene according to claims 1 to 2, by the known method of resolution of the diastereomeric salts using optically active acids and then releasing the enantiomers, characterised in that dextrorotatory dibenzoyl tartaric acid is used as optically active acid.

5. Pharmaceutical preparations, characterised in that they contain a compound according to claims 1 to 2 as an active substance in admixture with conventional pharmaceutical auxiliaries and carriers.

**Claims for the contracting State: AT**

1. Process for the production of (+)-2-[1-(2,6-dichlorophenoxy)-ethyl] -1,3-diazacyclopent-2-ene and the pharmaceutically acceptable acid addition salts thereof, characterised in that a (+)-2-hydroxy-propionic acid-$C_{1-4}$-alkylester is reacted with thionylchloride in the presence of catalytic quantities of dimethylformamide with configuration reversal, to produce a (−)-2-chloropropionic acid-$C_{1-4}$-alkyl ester which is converted into (+)-2-(2,6-dichlorophenoxy)- propionic acid-$C_{1-4}$ alkyl ester with a further configuration reversal, by etherification with 2,6-dichlorophenol in the presence of a base at a temperature in the range of from 60 to 150 °C, in a suitable organic solvent, this alkylester being used for the production of the (+)-1,3-diazacyclopentene derivative according to known methods.

2. Process for the production of (+)-2-[1-(2,6-dichlorophenoxy) -ethyl] -1,3-diazacyclopent-2-ene and the pharmaceutically acceptable acid addition salts thereof, by the known method of resolution of the diastereomeric salts using optically active acids and then releasing the enantiomers, characterised in that dextrorotatory dibenzoyl tartaric acid is used as optically active acid.